(19) Europäisches Patentamt
European Patent Office
Office européen des brevets

(11) Veröffentlichungsnummer : **0 171 645**
**B1**

(12) # EUROPÄISCHE PATENTSCHRIFT

(45) Veröffentlichungstag der Patentschrift : **30.11.88**

(51) Int. Cl.[4] : **C 07 D311/18**, C 07 D311/16, A 61 K 31/495

(21) Anmeldenummer : **85109016.7**

(22) Anmeldetag : **19.07.85**

(54) **Neue 2H-1-Benzopyran-2-on-Derivate, Verfahren zu ihrer Herstellung und Arzneimittel, die diese Verbindungen enthalten.**

(30) Priorität : **28.07.84 DE 3427985**

(43) Veröffentlichungstag der Anmeldung : **19.02.86 Patentblatt 86/08**

(45) Bekanntmachung des Hinweises auf die Patenterteilung : **30.11.88 Patentblatt 88/48**

(84) Benannte Vertragsstaaten : **AT BE CH DE FR GB IT LI LU NL SE**

(56) Entgegenhaltungen :
**DE-A- 2 123 924**
**CHEMICAL ABSTRACTS, Band 88, 1978, Seite 35, Nr. 44918b, Columbus, Ohio, US; E. ROESCH et al.: "Prostaglandin antagonism and antianaphylactic drug activity" & MONOGR. ALLERGY 1977, 12(Mediators Immed. Type Inflammatory React., Symp. Coll. Int. Allergol. 11th), 164-8**
**CHEMICAL ABSTRACTS, Band 91, 1979, Seite 67, Nr. 168618k, Columbus, Ohio, US; E. GONSIOR et al.: "Protective antiallergic effects of a new coumarin compound (BM15,100) in experimental asthma" & INT. J. CLIN. PHARMACOL. BIOPHARM. 1979, 17(7), 283-9**

(73) Patentinhaber : **BOEHRINGER MANNHEIM GMBH**
**Sandhofer Strasse 116**
**D-6800 Mannheim 31 (DE)**

(72) Erfinder : **Witte, Ernst-Christian, Dr. rer. nat.**
**Beethovenstrasse 2**
**D-6800 Mannheim 1 (DE)**
Erfinder : **Neubert, Peter, Dr. rer. nat.**
**Telemannstrasse 5**
**D-6940 Weinheim (DE)**
Erfinder : **Roesch, Androniki, Dr. med.**
**Werderstrasse 44**
**D-6800 Mannheim 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 171 645 B1

Jouve, 18, rue St-Denis, 75001 Paris, France

**Beschreibung**

Gegenstand der vorliegenden Erfindung sind neuartige 2H-1-Benzopyran-2-on-Derivate der allgemeinen Formel I

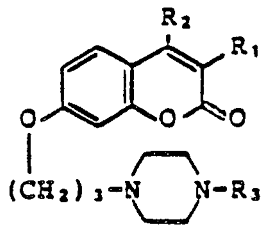

(I)

in der entweder

$R_1$ eine $C_1$-$C_6$-Alkylgruppe, die gegebenenfalls durch Phenyl substituiert ist, das durch $C_1$-$C_6$-Alkyl, $C_1$-$C_6$-Alkoxy, Hydroxy oder Halogen substituiert sein kann, und

$R_2$ Trifluormethyl, Cyano, Hydroxymethyl, $C_1$-$C_6$-Alkoxymethyl, $C_1$-$C_6$-Acyloxymethyl, Halogenmethyl, Aminomethyl, Mono- oder Dialkylaminomethyl, $C_1$-$C_6$-Acyl, Carboxyl, $C_1$-$C_6$-Alkoxycarbonyl oder Carbamoyl, das durch $C_1$-$C_6$-Alkyl-mono- oder disubstituiert sein kann,

oder

$R_1$ Trifluormethyl, Cyano, Hydroxymethyl, Alkoxymethyl, $C_1$-$C_6$-Acyloxymethyl, Halogenmethyl, Aminomethyl, Mono- oder Dialkylaminomethyl, $C_1$-$C_6$-Acyl, Carboxyl, $C_1$-$C_6$-Alkoxycarbonyl oder Carbamoyl, das durch $C_1$-$C_6$-Alkyl-mono- oder disubstituiert sein kann,

$R_2$ eine $C_1$-$C_6$-Alkylgruppe, die gegebenenfalls durch Phenyl substituiert ist, das durch $C_1$-$C_6$-Alkyl, $C_1$-$C_6$-Alkoxy, Hydroxy oder Halogen substituiert sein kann, und

$R_3$ eine Phenyl- oder Benzylgruppe, die durch $C_1$-$C_6$-Alkyl, $C_1$-$C_6$-Alkoxy, Hydroxy oder Halogen substituiert sein kann,

bedeuten, sowie deren pharmakologisch verträglichen Salze.

Als niedere Alkylgruppen $R_1$ oder $R_2$ sowie als Substituenten der Phenylgruppen kommen solche mit 1-6, vorzugsweise mit 1-4 C-Atomen, insbesondere Methyl und Ethyl, in Frage.

Niedere Alkoxy-Substituenten enthalten 1-6, vorzugsweise 1-4 C-Atome ; bevorzugt sind Methoxy und Ethoxy. Acyloxymethyl bedeutet insbesondere Benzoyloxy-, Pivaloyloxy- und Acetyloxy-methyl.

Acyl enthält 1-6 C-Atome, bevorzugt sind die Gruppen Formyl, Acetyl und Propionyl. Halogen bedeutet in allen Fällen Fluor, Chlor und Brom ; bevorzugt ist Chlor.

Bevorzugte Substituenten am Aminogruppen (auch von Amiden) sind Methyl und Ethyl. Als Alkoxycarbonyl-Gruppen sind bevorzugt Methoxy- und Ethoxycarbonyl. Die neuen Verbindungen wirken auch nach oraler und inhalativer Gabe stark hemmend auf allergische Reaktionen der Haut und der Bronchien. Sie unterdruecken die Antigen- oder anti-IgE-bedingte Freisetzung von Histamin und anderen Mediatoren wie Proteinasen aus verschiedenen Zellen, z. B. Human-Leukozyten. Die Verbindungen haben darueber hinaus antagonistische Eigenschaften gegenueber Mediatoren, insbesondere gegenueber Histamin.

Aufgrund dieser Eigenschaften koennen die Verbindungen auch als Entzuendungshemmer angesehen werden.

Gegenüber den in DE-A 2 123 924 beschriebenen antiallergisch und antiinflammatorisch wirksamen Verbindungen, insbesondere 1-(4-Chlorphenyl)-4-[3-(4,4-dimethyl-cumarin-7-yl-oxy) propyl]-piperazin zeigen die erfindungsgemäßen Verbindungen hinsichtlich der Hemmung von Antigen induzierten Bronchospasmen in passiv sensibilisierten Meerschweinchen eine überlegene Wirkung, was nicht zu erwarten war.

Das Verfahren zur Herstellung von Verbindungen der allgemeinen Formel I ist dadurch gekennzeichnet, daß man entweder

a) Verbindungen der allgemeinen Formel II

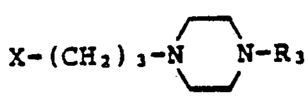

(II)

in der $R_3$ die oben angegebene Bedeutung hat und X einen reaktiven Rest darstellt, mit Verbindungen der allgemeinen Formel III

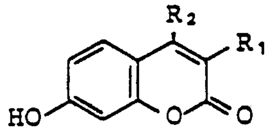

(III)

in der $R_1$ und $R_2$ die oben angegebene Bedeutung haben, in an sich bekannter Weise kondensiert, oder

b) Verbindungen der allgemeinen Formel IV

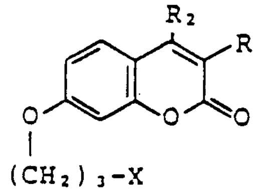

(IV)

in der $R_1$, $R_2$ und X die oben angegebene Bedeutung haben, mit Verbindungen der allgemeinen Formel V

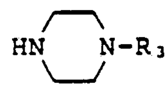

(V)

in der $R_3$ die oben angegebene Bedeutung hat, in an sich bekannter Weise kondensiert, oder

c) fuer den Fall, daß $R_3$ ein gegebenenfalls substituierter Benzylrest ist, Verbindungen der allgemeinen Formel VI

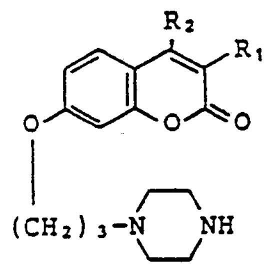

(VI)

in der $R_1$ und $R_2$ die oben angegebene Bedeutung haben, mit Verbindungen der Formel $X$-$R_3$, in der $R_3$ und X die oben angegebene Bedeutung haben, in an sich bekannter Weise kondensiert, worauf man die erhaltenen Produkte gewuenschtenfalls in ihre physiologisch vertraeglichen Salze ueberfuehrt.

Als reaktiver Rest X kommt insbesondere Halogen infrage.

Andererseits kann man die Verbindungen der allg. Formel I auch dadurch herstellen, daß man eine Verbindung der allg. Formel VII

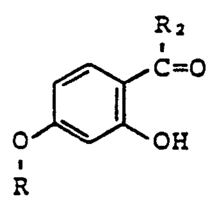

(VII)

in welcher R ein Wasserstoffatom, eine leicht zu entfernende Schutzgruppe (wie z. B. Tetrahydropyranyl, Benzyl oder Methyl) oder aber die Gruppe VIII

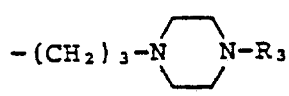

(VIII)

darstellt, mit einer Verbindung der allg. Formel IX

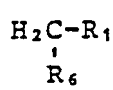

(IX)

in welcher $R_6$ eine Carboxylfunktion, eine Ester- oder Nitrilgruppe oder die Funktion

$$—CO—O—CO—CH_2R_1$$

bedeutet, zur Kondensation bringt, gegebenenfalls die Schutzgruppe entfernt und das entstehende Phenol gemaeß a) oder b) umsetzt.

Verbindungen der allgemeinen Formel I, in denen $R_1$ eine elektronegative Gruppe wie z. B. die Nitrilfunktion darstellt und $R_2$ eine niedere Alkylgruppe oder eine ggf. substituierte Benzylgruppe bedeutet, lassen sich aus Verbindungen der allgemeinen Formel X

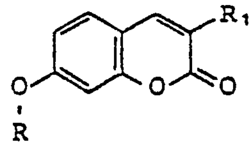

(X)

in welcher R die oben angegebene Bedeutung hat, durch Umsetzen mit Diazoalkanen oder mit ggf. im Kern substituierten Phenyldiazomethan herstellen. Es handelt sich hier also um eine Alkylierung bzw. Benzylierung in der 4-Stellung des Cumarin-Ringes.

Verbindungen der allg. Formel I sind auch dadurch zugaenglich, daß man Veraenderungen an den Substituenten $R_1$ bzw. $R_2$ vornimmt.

Solche Veraenderungen koennen bewirkt werden

1. durch Oxidations- oder Reduktionsreaktionen
2. durch Hydrolyse- oder Alkoholysereaktionen
3. durch Alkylierungs- oder Acylierungsreaktionen.

Beispiele fuer solche Veraenderungen sind die Reduktion einer Carboxylfunktion zur Hydroxymethylgruppe bzw. die Oxidation einer Hydroxymethylgruppe zur Carboxylfunktion, aber auch z. B. die Oxidation einer Methylgruppe mittels N-Halogenimiden zur Halogenmethylgruppe. Zu 2. waere als Beispiel die Hydrolyse der Nitrilfunktion zum Carbonamid und weiter zur Carbonsaeure, oder auch die Alkoholyse der Nitrilgruppe zum Carbonsaeureester zu nennen.

Als Alkylierungen bzw. Acylierungen gemaeß 3. seien beispielhaft angefuehrt die Veretherung einer Hydroxymethylgruppe durch alkylierende Agenzien oder die Bildung eines Carbonamids durch Acylierung der Aminomethylfunktion.

Die unter a-c formulierten Reaktionen werden saemtlich nach an sich bekannten Verfahren durchgefuehrt. So wird z. B. in der US-A-3 311 636 die Umsetzung von Hydroxysalicylaldehyden und in der US-A-3 053 679 die Umsetzung von 4-Methyl-5,7-dihydroxycumarinen mit Aminoalkylhalogeniden in Gegenwart von Alkalihydroxid beschrieben.

Um die bei den Reaktionen a-c frei werdende Saeure HX abzufangen, wird vorzugsweise in Gegenwart von saeurebindender Agenzien, z. B. Alkali- oder Erdalkali-carbonaten, Alkali-hydrogencarbonaten, Alkali- oder Erdalkali-hydroxiden oder Alkali-alkoholaten gearbeitet. Fuer die Aminalkylierung sind auch tertiaere Amine als Saeureakzeptoren geeignet.

Als Reaktionsmedium werden bevorzugt inerte Loesungsmittel wie z. B. Aceton, Butanon, Dimethylformamid, niedere Alkohole, fuer die Aminalkylierung auch zyklische Ether wie Tetrahydrofuran oder Dioxan, angewendet.

Fuer die an sich bekannten Ringschlußreaktionen werden sowohl saure als auch alkalische Kondensationsmittel angewandt. Als saures Kondensationsmittel sei z. B. gasfoermiger Chlorwasserstoff (evtl. in Gegenwart von Lewis-Saeuren wie z. B. Zinkchlorid) genannt. Als basische Kondensationsmittel werden z. B. Alkali- und Erdalkali-hydroxide, Alkali-alkoholate und Alkalisalze von Carbonsaeuren eingesetzt.

Die Kondensationsreaktionen werden zweckmaeßig in einem fluessigen Reaktionsmedium wie z. B. Eisessig (fuer saure Kondensationen) oder in Wasser bzw. Alkoholen (fuer basische Kondensationen) ausgefuehrt. In einzelnen Faellen werden solche Kondensationen aber auch durch Zusammenschmelzen der Reaktionskomponenten bei Temperaturen um 220 °C vorgenommen, wobei sich der Zusatz von katalytischen Mengen eines Hilfsstoffes wie z. B. Natriumacetat als foerderlich erweist.

Die Alkylierung bzw. Benzylierung mit Diazoalkanen bzw. Phenyldiazomethan wurde zuerst von Clinging, Dean, Houghton und Park in Tetrahedron Letters 15 (1976), 1227-1228 beschrieben. Die Umsetzung erfolgt bei Temperaturen zwischen 0 und 20 °C in einem Gemisch aus Tetrahydrofuran und Ether.

Für die skizzierten Umwandlungen funktioneller Gruppen $R_1$ bzw. $R_2$ in andere funktionelle Gruppen werden Standardverfahren der organischen Chemie eingesetzt.

Es ist offensichtlich, daß man alkylierbare funktionelle Gruppen $R_1$ bzw. $R_2$ zunaechst durch Schutzgruppen blockieren muß, wenn man Alkylierungen am Hydroxycumarin (Verfahren a) oder am Piperazinring (Verfahren b und c) vornehmen will. Nach erfolgter Umsetzung gemaeß a, b oder c wird die Schutzgruppe wieder entfernt.

Zur Herstellung von Salzen mit pharmakologisch vertraeglichen organischen oder anorganischen Saeuren, wie z. B. Salzsaeure, Schwefelsaeure, Phosphorsaeure, Milchsaeure, Citronensaeure oder Alkylsulfonsaeure koennen die Substanzen mit den entsprechenden Saeuren umgesetzt werden.

Zur Herstellung von Arzneimitteln werden die Substanzen I in an sich bekannter Weise mit geeigneten pharmazeutischen Traegersubstanzen, Aroma-, Geschmacks- und Farbstoffen gemischt und

beispielsweise als Tabletten oder Dragées ausgeformt oder unter Zugabe entsprechender Hilfsstoffe in Wasser oder Oel, wie z. B. Olivenoel, suspendiert oder geloest. Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat — oder Borat-Puffer, Ethanol, Dimethylsulfoxid, Komplexbildner (wie Ethylendiamintetraessig-säure), hochmolekulare Polymere (wie flüssiges Polyethylenoxid) zur Viskositätsregelung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z. B. mit pulverförmigen, physiologisch verträglichen Verdünnungsmitteln bzw. üblichen Salbengrundlagen vermischt.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

In den nachfolgenden Beispielen ist das erfindungsgemaeße Verfahren naeher erlaeutert :

Bevorzugt außer den in den Beispielen genannten Verbindungen sind die folgenden :

1. 3-Bromomethyl-7-<3-<4-[(4-chlorophenyl)methyl]-1-piperazinyl>propoxy-4-methyl-2H-1-benzopyran-2-on
2. 3-Aminomethyl-7-<3-<4-[(4-chlorophenyl)methyl]-1-piperazinyl>propoxy>-4-methyl-2H-1-benzopyran-2-on
3. 7-<3-<4-[(Chlorophenyl)methyl]-1-piperazinyl>propoxy>-3-diethylamino methyl-4-methyl-2H-1-benzopyran-2-on
4. 7-<3-<4-[(4-Chlorophenyl)methyl]-1-piperazinyl>propoxy>-3-methoxymethyl-4-methyl-2H-1-benzopyran-2-on
5. 7-<3-<4-[(4-Chlorophenyl)methyl]-1-piperazinyl>propoxy>-4-methyl-3-trifluoromethyl-2H-1-benzopyran-2-on
6. 7-<3-<4-[(4-Chlorophenyl)methyl]-1-piperazinyl>propoxy>–3-cyano-4-phenylmethyl-2H-1-benzopyran-2-on
7. 7-<3-<4-[(4-Chlorophenyl)methyl]-1-piperazinyl>propoxy>-4-phenylmethyl-2H-1-benzopyran-2-on-3-carbonsaeure
sowie deren Ethylester, Amid und Diethylamid
8. 4-[(4-Chlorophenyl)methyl]-7-<3-<4-[(4-chlorophenyl)-methyl]-1-piperazinyl>propoxy>-3-cyano-2H-1-benzopyran-2-on
9. 4-[(4-Chlorophenyl) methyl]-7-<3-<4-[(4-chlorophenyl)-methyl]-1-piperazinyl>propoxy>-2H-1-benzopyran-2-on-3-carbonsaeure
sowie deren Ethylester, Amid und Diethylamid
10. 7-<3-<4-[(4-Chlorophenyl)methyl]-1-piperazinyl>propoxy>-3-phenylmethyl-2H-1-benzopyran-2-on-4-carbonsaeure
sowie deren Amid.
11. 7-<3-<4-[(4-Chlorophenyl)methyl]-1-piperazinyl>propoxy>-4-hydroxymethyl-3-phenylmethyl-2H-1-benzopyran-2-on
12. 7-<3-<4-[(4-Chlorophenyl)methyl]-1-piperazinyl>propoxy>-4-methoxymethyl-3-phenylmethyl-2H-1-benzopyran-2-on
13. 3-[(4-Chlorophenyl)methyl]-7-<3-<4-[(4-Chlorophenyl)-methyl]-1-piperazinyl>propoxy>-2H-1-benzopyran-2-on-4-carbonsaeure
sowie deren Ethylester und Amid
14. 3-[(4-Chlorophenyl)methyl]-7-<3-<4-[(4-chlorophenyl)-methyl]-1-piperazinyl>propoxy>-4-hydroxymethyl-2H-1-benzopyran-2-on
15. 3-[(4-Chlorophenyl)methyl]-7-<3-<4-[(4-chlorophenyl)-methyl]-1-piperazinyl>propoxy>-4-methoxymethyl-2H-1-benzopyran-2-on

## Beispiel 1

7-<3-<4-[(4-Chlorophenyl)methyl]-1-piperazinyl>propoxy>-3-methyl-2H-1-benzopyran-2-on-4-carbonsaeure-ethylester

Man haelt eine Suspension aus 10.5 g (42 mmol) 7-Hydroxy-3-methyl-2H-1-benzopyran-2-on-4-carbonsaeure-ethylester, 230 ml Butanon und 18.0 g (130 mmol) pulverisiertem, wasserfreiem Kaliumcarbonat zwei Stunden auf Rueckflußtemperatur, kuehlt ab, gibt eine Spatelspitze Kaliumiodid zu und tropft nun eine Loesung aus 100 ml Butanon und 13.3 g (46 mmol) 3-<4-[(4-Chlorophenyl)methyl]-1-piperazinyl>propylchlorid zu. Anschließend wird 16 Stunden auf Rueckflußtemperatur gehalten. Man kuehlt dann etwas, saugt noch warm ab und dampft das Filtrat ein. Der Rueckstand wird in Ether geloest, die etherische Loesung mit Aktivkohle behandelt, filtriert und wieder eingedampft. Man loest nun die Rohbase in warmen Ethanol, gibt die doppelt molare Menge Maleinsaeure zu, kuehlt, saugt das Salz ab und kristallisiert es aus Ethanol um. Ausb. 27.0 g (87 % d. Th.) Di-maleinat mit dem Schmp. 175-176 °C. Die freie Base schmilzt bei 75 °C (Ethanol).

In Analogie dazu wird dargestellt

a) 7-<3-<4-[(4-Chlorophenyl)methyl]-1-piperazinyl>propoxy>-3-phenylmethyl-2H-1-benzopyran-2-on-4-carbonsaeure-ethylester

aus 7-Hydroxy-3-phenylmethyl-2H-1-benzopyran-2-on-4-carbonsaeure-ethylester und 3-<4-[(4-Chlorophenyl)methyl]-1-piperazinyl>propylchlorid

Beispiel 2

7-<3-<4-[(4-Chlorophenyl)methyl]-1-piperazinyl>propoxy>-3-methyl-2H-1-benzopyran-2-on-4-carbonsaeure

Man ruehrt ein Gemisch aus 13.5 g (27 mmol) des voranstehend beschriebenen Ethylesters (in Form der freien Base), 50 ml Ethanol und 25 ml 2 N-NaOH drei Stunden bei Raumtemperatur, saeuert mit conc. HCl an und engt im Vakuum etwas ein. Dann wird abgesaugt und das Rohprodukt aus sehr verduennter Salzsaeure umkristallisiert.

Ausb. 11.8 g (80 % d. Th.) Dihydrochlorid mit dem Schmp. 238-241 °C (Z).

Beispiel 3

7-<3-<4-[(4-Chlorophenyl)methyl]-1-piperazinyl>propoxy>-4-hydroxymethyl-3-methyl-2H-1-benzopyran-2-on

Ein Gemisch aus 1.7 g (3 mmol) 7-<3-<4-[(4-Chlorophenyl)-methyl]-1-piperazinyl>propoxy>-3-methyl-2H-1-benzopyran-2-on-4-carbonsaeure-dihydrochlorid, 0.98 g (8.7 mmol) abs. Triethylamin und 20 ml abs. Tetrahydrofuran wird 30 min bei Raumtemperatur geruehrt. Dann kuehlt man und tropft bei einer Innentemperatur von —5 bis —10 °C eine Loesung aus 0.38 g (3.5 mmol) Chlorameisensaeureethylester und 10 ml abs. Tetrahydrofuran zu, laeßt 30 min bei —5 °C reagieren und saugt schließlich unter Feuchtigkeitsausschluß das ausgefallene Triethylaminhydrochlorid ab. Das Filtrat wird bei + 10 °C zu einer Suspension aus 93 mg (2.5 mmol) Natriumborhydrid und 1.5 ml Wasser getropft.

Man ruehrt nun zwei Stunden bei Raumtemperatur, dampft anschließend im Vakuum ein und versetzt den Rueckstand mit einem Gemisch aus Eis und Natriumhydrogencarbonatloesung. Dann wird mehrmals mit Essigester extrahiert, der Extrakt mit Natriumsulfat getrocknet und schließlich eingedampft. Nach Umkristallisieren aus Ethanol 0.84 g (59 % d. Th.) Base mit dem Schmp. 135 °C. Dihydrochlorid : Schmp. 241 °C (Methanol).

Beispiel 4

7-<3-<4-[(4-Chlorophenyl)methyl]-1-piperazinyl>propoxy>-3-methyl-4-trifluormethyl-2H-1-benzopyran-2-on

1. Man erhitzt ein Gemisch aus 18.1 g (87.8 mmol) 1-(2,4-Dihydroxyphenyl)-2,2,2-trifluoro-ethanon, 23.5 g (180.6 mmol) Propionsaeure-anhydrid und 6.0 g (62.5 mmol) Natriumpropionat 8 Stunden auf 190 °C, kuehlt dann ab, loest in Ethanol und versetzt mit conc. Ammoniak bis zur stark alkalischen Reaktion. Nach 30 min Stehen wird mit 2N-HCl angesaeuert und mit Methylenchlorid extrahiert. Man trocknet den Extrakt mit Natriumsulfat, dampft ein und kristallisiert den Rueckstand aus Toluol um. Ausbeute : 9.9 g (46 % d. Th.) 7-Hydroxy-3-methyl-4-trifluormethyl-2H-1-benzopyran-2-on mit dem Schmp. 161-162 °C.

2. Die Titelverbindung erhaelt man durch Umsetzen von 7-Hydroxy-3-methyl-4-trifluormethyl-2H-1-benzopyran-2-on mit 3-<4-[(4-Chlorophenyl)methyl]-1-piperazinyl>propylchlorid in Analogie zu Beispiel 1. Ausb. 12.4 g (54 % d. Th.) Dihydrochlorid, Schmp. 260 °C (Z).

Beispiel 5

In Analogie zu Beispiel 1 werden dargestellt :

Aus 7-Hydroxy-4-methyl-2H-1-benzopyran-2-on-3-carbonsaeure-ethylester und

a) 3-<4-[(4-Chlorophenyl)methyl]-1-piperazinyl>propylchlorid die Verbindung

7-<3-<4-[(4-Chlorophenyl)methyl]-1-piperazinyl>propoxy>-4-methyl-2H-1-benzopyran-2-on-3-carbonsaeure-ethylester

Ausbeute : 83 % d. Th. Dihydrochlorid
Schmp. : 245 °C (Z) (waeßr. Ethanol)

b) 3-<4-[(2-Chlorophenyl)methyl]-1-piperazinyl>propylchlorid die Verbindung

7-<3-<4-[(2-Chlorophenyl)methyl]-1-piperazinyl>propoxy>-4-methyl-2H-1-benzopyran-2-on-3-carbonsaeure-ethylester
c) und 3-(4-Phenyl-1-piperazinyl)propylchlorid die Verbindung

4-Methyl-7-<3-[4-phenyl-1-piperazinyl]propoxy>-2H-1-benzopyran-2-on-3-carbonsaeure-ethylester

Beispiel 6

In Analogie zu Beispiel 1 wird dargestellt :
7-<3-<4-[(4-Chlorophenyl)methyl]-1-piperazinyl>propoxy>-3-cyano-4-methyl-2H-1-benzopyran-2-on
Durch Umsetzen von 3-Cyano-7-hydroxy-4-methyl-2H-1-benzopyran-2-on mit 3-<4-[(4-Chlorophenyl)methyl]-1-piperazinyl>propylchlorid.
Ausbeute : 61 % d. Th. Di-maleinat
Schmp. : 170-171 °C (Z) (Methanol).

Beispiel 7

Durch Hydrolyse der entsprechenden Ethylester analog Beispiel 2 werden hergestellt :
a) 7-<3-<4-[(4-Chlorophenyl)methyl]-1-piperazinyl>propoxy>-4-methyl-2H-1-benzopyran-2-on-3-carbonsaeuere
Ausb. 70 % d. Th. Dihydrochlorid-monohydrat Schmp. 252-253 °C (Z) (waeßr. Ethanol).

b) 7-<3-<4-[(2-Chlorophenyl) methyl]-1-piperazinyl>propoxy>-4-methyl-2H-1-benzopyran-2-on-3-carbonsaeure

c) 4-Methyl-7-[3-(4-phenyl-1-piperazinyl) propoxy]-2H-1-benzopyran-2-on-3-carbonsaeure

d) 7-<3-<4-[(4-Chlorophenyl) methyl]-1-piperazinyl>propoxy>-4-phenylmethyl-2H-1-benzopyran-2-on-3-carbonsäure als Dihydrochlorid-monohydrat.
Schmp. 243-246 °C, Ausb. 69 % d. Th.

Beispiel 8

7-<3-<4-[(4-Chlorophenyl) methyl]-1-piperazinyl>propoxy>-4-methyl-2H-1-benzopyran-2-on-3-carbonsaeureamid

1. Man ruehrt ein Gemisch aus 12.0 g (22 mmol) 7-<3-<4-[(4-Chlorophenyl) methyl]-1-piperazinyl>propoxy>-4-methyl-2H-1-benzopyran-2-on-3-carbonsaeure-dihydrochlorid, 100 ml abs. Chloroform und 36 g (0.3 mol) Thionylchlorid 4 Stunden bei 60 °C, wobei sich ein dicker Kristallbrei bildet. Nach Stehen ueber Nacht wird zur Trockne eingedampf und der Rueckstand mit abs. Ether verruehrt. Nach Absaugen und Trocknen erhaelt man 12.0 g (97 % d. Th.) 7-<3-<4-[(4-Chlorophenyl) methyl]-1-piperazinyl>propoxy>-4-methyl-2H-1-benzopyran-2-on-3-carbonsaeurechlorid in Form des Dihydrochlorids. Schmp. 257-259 °C.

2. Die Titelverbindung erhaelt man durch langsames Eintragen des Saeurechlorids in fluessiges Ammoniak, Abdampfen des Ammoniaks und Aufarbeiten des Rueckstandes. Dieser wird mit verd. Ammoniakwasser behandelt. Man extrahiert mit Methylenchlorid, waescht die organische Phase mit Wasser und trocknet mit $Na_2SO_4$. Dann wird das Methylenchlorid abgedampft, der Rueckstand in Ether geloest und durch Zugabe von chlorwasserstoffhaltigem Ether das Dihydrochlorid ausgefaellt. Nach dem Umkristallisieren aus waeßrigem Methanol erhaelt man das reine Dihydrochlorid in 64 % Ausbeute. Schmp. 256-258 °C.
Eine Variante dieses Verfahrens besteht darin, daß man das Saeurechlorid nicht in fluessiges Ammoniak, sondern in ein eiskaltes Gemisch aus Dioxan und konz. Ammoniakwasser eintraegt und entsprechend aufarbeitet.
Nach dieser Variante wird hergestellt :

a) 7-<3-<4-[(4-Chlorophenyl) methyl]-1-piperazinyl>propoxy>-3-methyl-2H-1-benzopyran-2-on-4-carbonsaeureamid

Beispiel 9

7-<3-<4-[(4-Chlorophenyl) methyl]-1-piperazinyl>propoxy>-4-methyl-2H-1-benzopyran-2-on-3-carbonsaeure-diethylamid

Zu einer eiskalten Loesung aus 7.8 g (107 mmol) Diethylamin und 30 ml abs. Tetrahydrofuran gibt man unter Ruehren portionsweise 6.0 g (107 mmol) des nach Beispiel 8 a) erhaltenen Saeurechlorids (in Form des Dihydrochlorids) und ruehrt weitere zwei Stunden. Dann wird eingedampft, der Eindampfrueckstand in Ethanol geloest und durch Zusetzen von chlorwasserstoffhaltigem Ether das Dihydrochlorid ausgefaellt. Nach Absaugen und Umkristallisieren aus Methanol, welches einige Prozente 2 N-HCl enthaelt, resultieren 4.65 g (73 % d. Th.) Diethylamiddihydrochlorid mit dem Schmp. 227-229 °C.

In Analogie dazu erhält man aus dem entsprechenden Säurechlorid und Diäthylamin die Verbindung

7-<3-<4-[(4-Chlorophenyl) methyl]-1-piperazinyl>propoxy>-4-phenylmethyl-2H-1-benzopyran-2-on-3-carbonsäure-diethylamid als Dihydrochlorid mit dem Schmp. 228-230 °C. Ausb. 57 % d. Th.

Beispiel 10

7-<3-<4-[(4-Chlorophenyl) methyl]-1-piperazinyl>propoxy>-3-hydroxymethyl-4-methyl-2H-1-benzopyran-2-on

Bei einer Temperatur von 0 bis + 5 °C traegt man unter starkem Ruehren portionsweise 5.0 g (9 mmol) des nach Beispiel 8 a) erhaltenen Saeurechlorids (in Form des Dihydrochlorids) in eine Suspension aus 5 g (130 mmol) Natriumborhydrid und 50 ml Wasser ein, wobei starke Schaumbildung auftritt. Anschließend wird eine Stunde bei 0 °C, eine weitere Stunde bei Raumtemperatur geruehrt, dann mit verd. HCl auf pH 4 gebracht. Das ausfallende, schmierige Produkt wird mit Natriumhydrogencarbonat-Loesung behandelt. Man extrahiert nun mit Methylenchlorid, trocknet die organische Phase mit Natriumsulfat und dampft ein. Nach Saeulenchromatographie (Kieselgel, Methylenchlorid + Methanol 19 + 1 Vol.) erhaelt man 2.1 g (52 % d. Th.) freie Base, deren Dihydrochlorid bei 256 °C schmilzt (aus waeßrigem Ethanol).

In analoger Weise werden aus den entsprechenden Saeurechloriden dargestellt :

a) 7-<3-<4-[(4-Chlorophenyl) methyl]-1-piperazinyl>propoxy>-3-hydroxymethyl-4-phenylmethyl-2H-1-benzopyran-2-on und

b) 4-[(4-Chlor-phenyl) methyl]-7-<3-<4-[(4-chlorophenyl) methyl]-1-piperazinyl>propoxy>-3-hydroxymethyl-2H-1-benzopyran-2-on

Beispiel 11

7-<3-<4-[(4-Chlorophenyl) methyl]-1-piperazinyl>propoxy>-4-phenylmethyl-2H-1-benzopyran-2-on-3-carbonsäure-ethylester

a) Ein Gemisch aus 20.0 g (63 mmol) 1-(2-Hydroxy-4-phenylmethyloxy-phenyl)-2-phenyl-ethanon, 52.8 g (0.33 mol) Methandicarbonsäure-diethylester und 6.0 g (50 mmol) Kalium-tert. butoxid wird 30 min bei 170 °C gerührt, dann kühlt man ab und löst in Toluol. Nach dem Abtrennen von Ungelöstem und Behandeln mit Kieselgel wird die Lösung eingedampft. Der kristalline Rückstand wird mit Ligroin gewaschen, dann aus Cyclohexan oder Essigester umkristallisiert. Man erhält 21.0 g (81 % d. Th.) 4-Phenylmethyl-7-phenylmethyloxy-2H-1-benzopyran-2-on-3-carbonsäure-ethylester mit dem Schmp. 117-118 °C.

b) Man löst 20.0 g (48.3 mmol) des nach a) erhaltenen Produkts in 200 ml Ethanol und 400 ml abs. Tetrahydrofuran, gibt ca. 1 g 5-proz. Palladiumkohle zu und hydriert 24 h in der Schüttelente bei Normaldruck. Nach Absaugen des Katalysators wird eingedampft, der kristalline Rückstand in heißem Essigester gelöst und Ungelöstes abgetrennt. Man versetzt nun mit Ligroin, saugt die ausfallenden Kristalle ab und kristallisiert aus Toluol um. Ausbeute 8.3 g (53 % d. Th.) 7-Hydroxy-4-phenylmethyl-2H-1-benzopyran-2-on-3-carbonsäure-ethylester mit dem Schmp. 150-152 °C.

c) Durch Umsetzen des nach b) erhaltenen Produktes mit 3-<4-[(4-Chlorophenyl) methyl]-1-piperazinyl>propylchlorid in Analogie zu Beispiel 1 erhält man in 73 % Ausbeute die Titelverbindung als Dihydrochlorid mit dem Schmp. 220-223 °C (wäßr. Ethanol).

Beispiel 12

7-<3-<4-[(4-Chlorophenyl) methyl]-1-piperazinyl>propoxy>-4-[(4-chlorophenyl) methyl]-2H-1-benzopyran-2-on-3-carbonsäure

a) Man erhitzt ein Gemisch aus 20.0 g (76 mmol) 1-(2.4-Dihydroxyphenyl)-2-(4-chlorophenyl)-ethanon, 105.6 g (0.66 mol) Methandicarbonsäure-diethylester und 10 g (89 mmol) Kalium-tert. butoxid ca. 10 min auf ca. 210 °C, so daß überschüssiger Methandicarbonsäure-diethylester abdestilliert. Es entsteht ein tiefroter Kristallbrei. Nach Abdestillieren von restlichem Methandicarbonsäureester i. Vak. versetzt man mit verd. Salzsäure, gibt Essigester zu und rührt, bis die Kristalle gelöst sind. Die organische Phase wird nun abgetrennt, mit $Na_2SO_4$ getrocknet und eingedampft. Man chromatographiert nun mittels Kieselgel/Toluol, bis alle Verunreinigungen die Säule verlassen haben und wäscht dann das gewünschte Produkt mit Ether aus der Säule aus. Nach Eindampfen und Umkristallisieren aus Ethanol erhält man 10.3 g (38 % d. Th.) 4-[(4-Chlorophenyl) methyl]-7-hydroxy-2H-1-benzopyran-2-on-3-carbonsäure-ethylester mit dem Schmp. 167-169 °C.

b) Durch Umsetzen des nach a) erhaltenen Produktes mit 3-<4-[(4-Chlorophenyl) methyl]-1-piperazinyl>propylchlorid in Analogie zu Beispiel 1 erhält man den Ethylester der Titelverbindung als Dihydrochlorid. Ausbeute 73 % d. Th., Schmp. 228-229 °C (wäßr. Ethanol).

c) Die Titelverbindung erhält man durch Verseifen des nach b) erhaltenen Ethylesters mit einem Gemisch aus 2N-NaOH und Ethanol in Analogie zu Beispiel 2. Ausbeute an Dihydrochlorid : 65 % d. Th., Schmp. 245-248 °C (wäßr. Aceton).

Beispiel 13

In Analogie zu Beispiel 8, Absatz 1, erhält man aus den entsprechenden Carbonsäuren durch Umsetzen mit Thionylchlorid in Chloroform die Verbindungen

7-<3-<4-[(4-Chlorophenyl) methyl]-1-piperazinyl>propoxy>-4-phenylmethyl-2H-1-benzopyran-2-on-3-carbonsäurechlorid als Dihydrochlorid mit dem Schmp. 280-283 °C. Ausbeute 92 % d. Th.

bzw.

7-<3-<4-[(4-Chlorophenyl) methyl]-1-piperazinyl>propoxy>-4-[(4-chlorophenyl) methyl]-2H-1-benzopyran-2-on-3-carbonsäurechlorid als Dihydrochlorid mit dem Schmp. 277-282 °C. Ausbeute 97 % d. Th.

Beispiel 14

In Analogie zu Beispiel 8, Variante a), wurde aus den entsprechenden Carbonsäurechloriden dargestellt :

7-<3-<4-[(4-Chlorophenyl) methyl]-1-piperazinyl>propoxy>-4-phenylmethyl-2H-1-benzopyran-2-on-3-carbonsäureamid als Dihydrochlorid mit dem Schmp. 185-189 °C. Das Dihydrochlorid-monohydrat schmilzt bei 169-173 °C. Ausbeute 72 % d. Th.

bzw.

7-<3-<4-[(4-Chlorophenyl) methyl]-1-piperazinyl>propoxy>-4-[(4-chlorophenyl) methyl]-2H-1-benzopyran-2-on-3-carbonsäureamid als Dihydrochlorid mit dem Schmp. 204-207 °C (wäßr. Aceton). Ausbeute 69 % d. Th.

Beispiel 15

Es wurden Tabletten hergestellt : Jede Tablette enthaelt 10 mg der Verbindung des Beispiels 1
Die Tabletten wurden gemäß der folgenden Rezeptur hergestellt :

| | |
|---|---|
| Verbindung des Beispiels 1 | 10 g |
| Lactose | 80 g |
| Staerke | 29 g |
| Magnesiumstearat | 1 g |

Vorstehende Verbindung wurde fein pulverisiert und mit Lactose und Staerke vermischt. Das Gemisch wurde in herkoemmlicher Weise granuliert. Magnesiumstearat wurde zu dem Granulat gegeben und das Gemisch zu 1 000 Tabletten mit einem Einzelgewicht von 0.12 g verpreßt.

**Patentansprüche**

1. 2H-1-Benzopyran-2-on-Derivate der allgemeinen Formel I

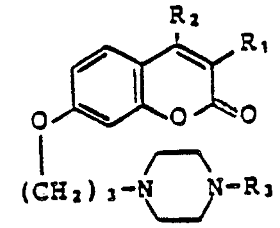

(I)

in der entweder
$R_1$ eine $C_1$-$C_6$-Alkylgruppe, die gegebenenfalls durch Phenyl substituiert ist, das durch $C_1$-$C_6$-Alkyl, $C_1$-$C_6$-Alkoxy, Hydroxy oder Halogen substituiert sein kann, und
$R_2$ Trifluormethyl, Cyano, Hydroxymethyl, $C_1$-$C_6$-Alkoxymethyl, $C_1$-$C_6$-Acyloxymethyl, Halogenmethyl, Aminomethyl, Mono- oder Dialkylaminomethyl, $C_1$-$C_6$-Acyl, Carboxyl, $C_1$-$C_6$-Alkoxycarbonyl oder Carbamoyl, das durch $C_1$-$C_6$-Alkyl-mono- oder disubstituiert sein kann,
oder
$R_1$ Trifluormethyl, Cyano, Hydroxymethyl, Alkoxymethyl, $C_1$-$C_6$-Acyloxymethyl, Halogenmethyl, Aminomethyl, Mono- oder Dialkylaminomethyl, $C_1$-$C_6$-Acyl, Carboxyl, $C_1$-$C_6$-Alkoxycarbonyl oder Carbamoyl, das durch $C_1$-$C_6$-Alkyl-mono- oder disubstituiert sein kann,
$R_2$ eine $C_1$-$C_6$-Alkylgruppe, die gebebenenfalls durch Phenyl substituiert ist, das durch $C_1$-$C_6$-Alkyl, $C_1$-$C_6$-Alkoxy, Hydroxy oder Halogen substituiert sein kann, und
$R_3$ eine Phenyl- oder Benzylgruppe, die durch $C_1$-$C_6$-Alkyl, $C_1$-$C_6$-Alkoxy, Hydroxy oder Halogen substituiert sein kann,
bedeuten, sowie deren pharmakologisch verträglichen Salze.

2. Verbindungen gemäß Anspruch 1, in denen $R_3$ 4-Chlorbenzyl darstellt und $R_1$ und $R_2$ die angegebene Bedeutung haben.

3. Verbindungen gemäß Anspruch 1 oder 2, in denen $R_1$ eine $C_1$-$C_6$-Alkyl-Gruppe, die durch Phenyl substituiert sein kann, $R_2$ einen Trifluormethyl-, Cyano-, Hydroxymethyl-, $C_1$-$C_6$-Alkoxymethyl-, Carboxyl- oder N,N'-$C_1$-$C_6$-Dialkylcarbamoyl-Rest darstellen und $R_3$ die angegebene Bedeutung hat.

4. Verfahren zur Herstellung von 2H-1-Benzopyran-2-on-Derivaten der allgemeinen Formel I

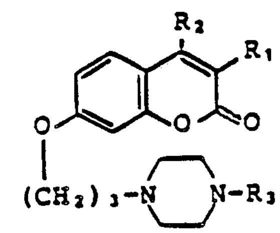

(I)

in der entweder
$R_1$ eine $C_1$-$C_6$-Alkylgruppe, die gegebenenfalls durch Phenyl substituiert ist, das durch $C_1$-$C_6$-Alkyl, $C_1$-$C_6$-Alkoxy, Hydroxy oder Halogen substituiert sein kann, und
$R_2$ Trifluormethyl, Cyano, Hydroxymethyl, $C_1$-$C_6$-Alkoxymethyl, $C_1$-$C_6$-Acyloxymethyl, Halogenmethyl, Aminomethyl, Mono- oder Dialkylaminomethyl, $C_1$-$C_6$-Acyl, Carbonyl, $C_1$-$C_6$-Alkoxycarbonyl oder Carbamoyl, das durch $C_1$-$C_6$-Alkyl-mono- oder disubstituiert sein kann,
oder
$R_1$ Trifluormethyl, Cyano, Hydroxymethyl, Alkoxymethyl, $C_1$-$C_6$-Acyloxymethyl, Halogenmethyl, Aminomethyl, Mono- oder Dialkylaminomethyl, $C_1$-$C_6$-Acyl, Carboxyl, $C_1$-$C_6$-Alkoxycarbonyl oder Carbamoyl, das durch $C_1$-$C_6$-Alkyl-mono- oder disubstituiert sein kann,
$R_2$ eine $C_1$-$C_6$-Alkylgruppe, die gegebenenfalls durch Phenyl substituiert ist, das durch $C_1$-$C_6$-Alkyl, $C_1$-$C_6$-Alkoxy, Hydroxy oder Halogen substituiert sein kann, und
$R_3$ eine Phenyl- oder Benzylgruppe, die durch $C_1$-$C_6$-Alkyl, $C_1$-$C_6$-Alkoxy, Hydroxy oder Halogen substituiert sein kann,
bedeuten, sowie deren pharmakologisch verträgliche Salze, dadurch gekennzeichnet, daß man entweder

a) Verbindungen der allgemeine Formel II

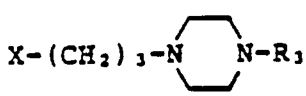

(II)

in der $R_3$ die oben angegebene Bedeutung hat und X einen reaktiven Rest darstellt, mit Verbindungen der allgemeinen Formel III

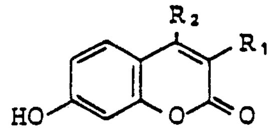

(III)

in der $R_1$ und $R_2$ die oben angegebene Bedeutung haben, in an sich bekannter Weise kondensiert, oder

b) Verbindungen der allgemeinen Formel IV

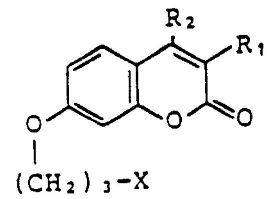

(IV)

in der $R_1$, $R_2$ und X die oben angegebene Bedeutung haben, mit Verbindungen der allgemeinen Formel V

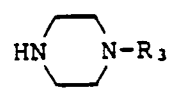

(V)

in der $R_3$ die oben angegebene Bedeutung hat, in an sich bekannter Weise kondensiert, oder

c) fuer den Fall, daß $R_3$ ein gegebenenfalls substituierter Benzylrest ist, Verbindungen der allgemeinen Formel VI

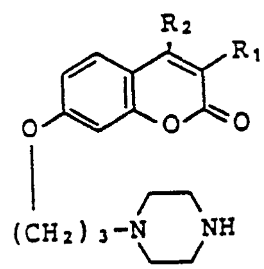

(VI)

in der $R_1$ und $R_2$ die oben angegebene Bedeutung haben, mit Verbindungen der Formel X-$R_3$, in der $R_3$ und X die oben angegebene Bedeutung haben, in an sich bekannter Weise kondensiert und anschließend gewünschtenfalls enthaltene Verbindungen der Formel I in andere Verbindungen der Formel I durch Oxidation oder Reduktion, Hydrolyse, Alkoholyse, Alkylierung oder Acylierung überführt sowie gegebenenfalls die enthaltenen Verbindungen in ein verträgliches Salz überführt.

5. Arzneimittel, enthaltend eine Verbindung gemäß Ansprüche 1-3 sowie übliche Träger- und Hilfsstoffe.

6. Verwendung von Verbindungen gemäß Anspruch 1-3 zur Herstellung von Arnzeimitteln zur Behandlung von allergischen Erkrankungen.

**Claims**

1. 2H-1-Benzopyran-2-one derivatives of the general formula I

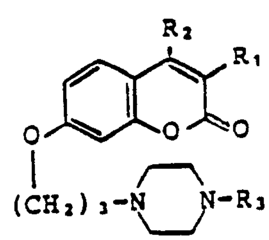

(I)

in which either

$R_1$ signifies a $C_1$-$C_6$-alkyl group, which is optionally substituted by phenyl, which can be substituted by $C_1$-$C_6$-alkyl, $C_1$-$C_6$-alkoxy, hydroxyl or halogen, and

$R_2$ signifies trifluoromethyl, cyano, hydroxymethyl, $C_1$-$C_6$-alkoxymethyl, $C_1$-$C_6$-acyloxymethyl, halogenomethyl, aminomethyl, mono- or dialkylaminomethyl, $C_1$-$C_6$-acyl, carboxyl, $C_1$-$C_6$-alkoxycarbonyl or carbamoyl, which can be mono- or di-substituted by $C_1$-$C_6$-alkyl ;

or

$R_1$ signifies trifluoromethyl, cyano, hydroxymethyl, alkoxymethyl, $C_1$-$C_6$-acyloxymethyl, halogenomethyl, aminomethyl, mono- or dialkylaminomethyl, $C_1$-$C_6$-acyl, carboxyl, $C_1$-$C_6$-alkoxycarbonyl or carbamoyl, which can be mono- or disubstituted by $C_1$-$C_6$-alkyl,

$R_2$ signifies a $C_1$-$C_6$-alkyl group, which is optionally substituted by phenyl, which can be substituted by $C_1$-$C_6$-alkyl, $C_1$-$C_6$-alkoxy, hydroxyl or halogen, and

$R_3$ signifies a phenyl or benzyl group which can be substituted by $C_1$-$C_6$-alkyl, $C_1$-$C_6$-alkoxy, hydroxyl or halogen ;

as well as their pharmacologically acceptable salts.

2. Compounds according to claim 1, in which $R_3$ represents 4-chlorobenzyl and $R_1$ and $R_2$ have the given meaning.

3. Compounds according to claim 1 or 2, in which $R_1$ represents a $C_1$-$C_6$-alkyl group, which can be substituted by phenyl, $R_2$ a trifluoromethyl, cyano, hydroxymethyl, $C_1$-$C_6$-alkoxymethyl, carboxyl or N,N'-$C_1$-$C_6$-dialkylcarbamoyl radical and $R_3$ has the given meaning.

4. Process for the preparation of 2H-1-benzopyran-2-one derivatives of the general formula I

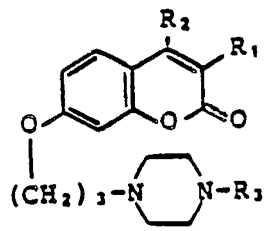

(I)

in which either

$R_1$ signifies a $C_1$-$C_6$-alkyl group, which is optionally substituted by phenyl, which can be substituted by $C_1$-$C_6$-alkyl, $C_1$-$C_6$-alkoxy, hydroxyl or halogen, and

$R_2$ signifies trifluoromethyl, cyano, hydroxymethyl, $C_1$-$C_6$-alkoxymethyl, $C_1$-$C_6$-acyloxymethyl, halogenomethyl, aminomethyl, mono- or dialkylaminomethyl, $C_1$-$C_6$-acyl, carboxyl, $C_1$-$C_6$-alkoxycarbonyl or carbamoyl, which can be mono- or di-substituted by $C_1$-$C_6$-alkyl ;

or

$R_1$ signifies trifluoromethyl, cyano, hydroxymethyl, alkoxymethyl, $C_1$-$C_6$-acyloxymethyl, halogenomethyl, aminomethyl, mono- or dialkylaminomethyl, $C_1$-$C_6$-acyl, carboxyl, $C_1$-$C_6$-alkoxycarbonyl or carbamoyl, which can be mono- or disubstituted by $C_1$-$C_6$-alkyl,

$R_2$ signifies a $C_1$-$C_6$-alkyl group which is optionally substituted by phenyl, which can be substituted by $C_1$-$C_6$-alkyl, $C_1$-$C_6$-alkoxy, hydroxyl or halogen, and

$R_3$ signifies a phenyl or benzyl group which can be substituted by $C_1$-$C_6$-alkyl, $C_1$-$C_6$-alkoxy, hydroxyl or halogen,

as well as of their pharmacologically acceptable salts, characterised in that one either

a) condenses in per se known manner compounds of the general formula II

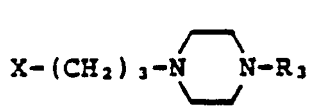

(II)

in which $R_3$ has the above-given meaning and X represents a reactive residue, with compounds of the general formula III

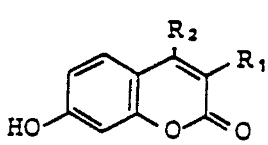

(III)

in which $R_1$ and $R_2$ have the above-given meaning, or

b) condenses in per se known manner compounds of the general formula IV

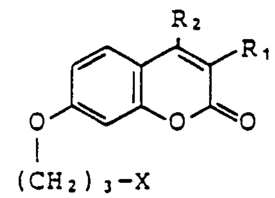

(IV)

in which $R_1$, $R_2$ and X have the above-given meaning, with compounds of the general formula V

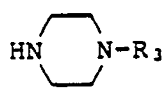

(V)

in which $R_3$ has the above-given meaning ; or

c) for the case that $R_3$ is an optionally substituted benzyl radical, condenses in per se known manner compounds of the general formula VI

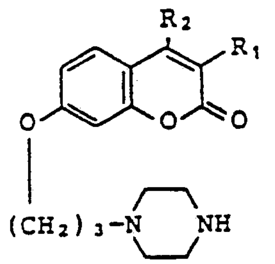

(VI)

in which $R_1$ and $R_2$ have the above-given meaning, with compounds of the formula $X-R_3$, in which $R_3$ and X have the above-given meaning ; and subsequently, if desired, converts the compound obtained of the formula I into other compounds of the formula I by oxidation or reduction, hydrolysis, alcoholysis, alkylation or acylation, as well as optionally converts the compounds obtained into a pharmacologically acceptable salt.

5. Medicaments containing a compound according to claims 1-3, as well as usual carrier and adjuvant materials.

6. Use of compounds according to claims 1-3 for the preparation of medicaments for the treatment of allergic diseases.

**Revendications**

1. Dérivés de 2H-1-benzopyrane-2-one de formule générale I

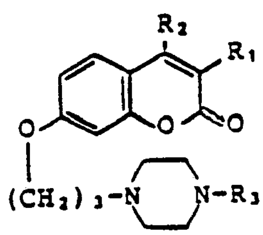

(I)

dans laquelle soit

$R_1$ représente un groupe alkyle en $C_1$-$C_6$, qui peut être éventuellement substitué par un groupe phényle, qui lui-même peut être substitué par un groupe alkyle en $C_1$-$C_6$, un groupe alcoxy en $C_1$-$C_6$, un groupe hydroxy ou un atome d'halogène, et

$R_2$ représente un groupe trifluorométhyle, cyano, hydroxyméthyle, alcoxyméthyle en $C_1$-$C_6$, acyloxyméthyle en $C_1$-$C_6$, halogénométhyle, aminométhyle, mono- ou di-alkylaminométhyle, acyle en $C_1$-$C_6$, carboxyle, alcoxycarbonyle en $C_1$-$C_6$ ou carbamoyle, qui peut être mono- ou di-substitué par un groupe alkyle en $C_1$-$C_6$

soit

$R_1$ représente un groupe trifluorométhyle, cyano, hydroxyméthyle, alcoxyméthyle, acyloxyméthyle en $C_1$-$C_6$, halogénométhyle, aminométhyle, mono- ou di-alkylaminométhyle, acyle en $C_1$-$C_6$, carboxyle, alcoxycarbonyle en $C_1$-$C_6$ ou carbamoyle, qui peut être mono- ou di-substitué par un groupe alkyle en $C_1$-$C_6$,

$R_2$ représente un groupe alkyle en $C_1$-$C_6$, qui peut être éventuellement substitué par un groupe phényle, qui lui-même peut être substitué par un groupe alkyle en $C_1$-$C_6$, un groupe alcoxy en $C_1$-$C_6$, un groupe hydroxy ou un atome d'halogène, et

$R_3$ représente un groupe phényle ou un groupe benzyle qui peut être substitué par un groupe alkyle en $C_1$-$C_6$, un groupe hydroxy ou un atome d'halogène,
ainsi que leurs sels pharmaceutiquement acceptables.

2. Composés selon la Revendication 1, dans lesquels $R_3$ représente le 4-chlorobenzyle et $R_1$ et $R_2$ ont la signification indiquée plus haut.

3. Composés selon les Revendications 1 ou 2 dans lesquelles $R_1$ représente un groupe alkyle en $C_1$-$C_6$ qui peut être substitué par un phényle, $R_2$ représente un groupe trifluorométhyle, cyano, hydroxyméthyle, alcoxyméthyle en $C_1$-$C_6$, carboxyle ou N,N'-dialkylcarbamoyle en $C_1$-$C_6$, et $R_3$ représente la signification indiquée plus haut.

4. Procédé de préparation des dérivés de 2H-1-benzopyran-2-on de formule générale I

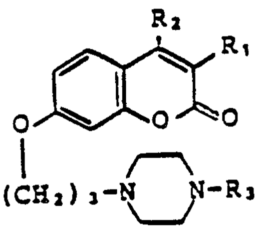

(I)

dans laquelle soit

$R_1$ représente un groupe alkyle en $C_1$-$C_6$, qui peut être éventuellement substitué par un groupe phényle, qui lui-même peut être substitué par un groupe alkyle en $C_1$-$C_6$, un groupe alcoxy en $C_1$-$C_6$, un groupe hydroxy ou un atome d'halogène, et

$R_2$ représente un groupe trifluorométhyle, cyano, hydroxyméthyle, alcoxyméthyle en $C_1$-$C_6$, acyloxyméthyle en $C_1$-$C_6$, halogénométhyle, aminométhyle, mono- ou di-alkylaminométhyle, acyle en $C_1$-$C_6$, carboxyle, alcoxycarbonyle en $C_1$-$C_6$ ou carbamoyle, qui peut être mono- ou di-substitué par un groupe alkyle en $C_1$-$C_6$,
soit

$R_1$ représente un groupe trifluorométhyle, cyano, hydroxyméthyle, alcoxyméthyle, acyloxyméthyle en $C_1$-$C_6$, halogénométhyle, aminométhyle, mono- ou di-alkylaminométhyle, acyle en $C_1$-$C_6$, carboxyle, alcoxycarbonyle en $C_1$-$C_6$ ou carbamoyle, qui peut être mono- ou di-substitué par un groupe alkyle en $C_1$-$C_6$,

$R_2$ représente un groupe alkyle en $C_1$-$C_6$, qui peut être éventuellement substitué par un groupe phényle, qui lui-même peut être substitué par un groupe alkyle en $C_1$-$C_6$, un groupe alcoxy en $C_1$-$C_6$, un groupe hydroxy ou un atome d'halogène, et

$R_3$ représente un groupe phényle ou un groupe benzyle qui peut être substitué par un groupe alkyle en $C_1$-$C_6$, un groupe hydroxy ou un atome d'halogène,
ainsi que leurs sels pharmaceutiquement acceptables, caractérisé en ce que l'on condense soit

a) Les composés de formule générale II

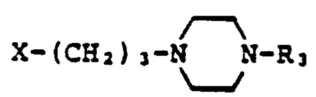

(II)

dans laquelle $R_3$ a la signification mentionnée plus haut et X représente un reste réactif, avec des composés de formule générale III

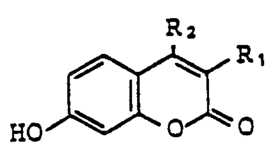

(III)

dans laquelle $R_1$ et $R_2$ ont la signification mentionnée plus haut, d'une manière connue en soi, ou bien

b) Les composés de formule générale IV

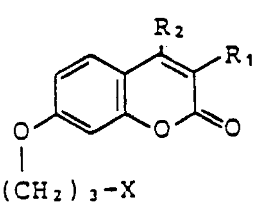

(IV)

dans laquelle $R_1$, $R_2$ et X ont la signification mentionnée plus haut, avec des composés de formule générale V

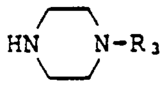

(V)

dans laquelle $R_3$ a la signification mentionnée plus haut, d'une manière connue en soi, ou bien

c) Dans le cas où $R_3$ est un reste benzyle éventuellement substitué, des composés de formule générale VI

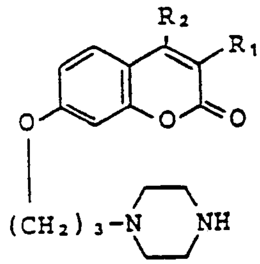

(VI)

dans laquelle $R_1$ et $R_2$ ont la signification mentionnée plus haut, avec des composés de formule X-$R_3$, dans laquelle $R_3$ et X ont la signification mentionnée plus haut, d'une manière connue en soi, après quoi on transforme le composé de formule I obtenu en d'autres composés de formule I par oxydation ou réduction, hydrolyse, alcoolyse, alkylation ou acylation, ainsi qu'éventuellement, on transforme le composé obtenu en un sel pharmaceutiquement acceptable.

5. Médicament contenant un composé selon les Revendications 1 à 3, ainsi que des matières de support et auxiliaires habituelles.

6. Application des composés selon les Revendications 1 à 3 pour la préparation de médicaments destinés au traitement d'affections allergiques.